# EUROPEAN PATENT SPECIFICATION

(11) **EP 3 684 454 B1**
(45) Date of publication and mention of the grant of the patent: **23.08.2023**
(21) Application number: 18857983.3
(22) Date of filing: 05.09.2018
(51) Int. Cl.: A61M 16/08, A61M 16/06, A61M 11/06

(54) **NEBULIZER ORIENTATION APPARATUS**
VERNEBLERORIENTIERUNGSVORRICHTUNG
APPAREIL D'ORIENTATION DE NÉBULISEUR

(30) Priority: 19.09.2017 ZA 201704891
(43) Date of publication of application: 29.07.2020
(73) Proprietor: Fish, Frank, 1501 Benoni (ZA)
(72) Inventor: BEN-ISRAEL, Karin-Ann, 2196 Johannesburg (ZA)
(74) Representative: Cremer & Cremer
(86) International application number: PCT/IB2018/056768
(87) International publication number: WO 2019/058199

(56) References cited:
- CN-U- 205 612 830
- ES-U- 1 045 126
- US-A- 5 357 945
- US-A1- 2005 092 325
- US-A1- 2007 049 841
- US-A1- 2012 285 452
- US-A1- 2014 166 010
- US-A1- 2017 007 797

## Description

### FIELD OF INVENTION

This invention relates to a nebulization means for use in the medical industry. Particularly, this invention relates to a nebulizer orientation apparatus for interposition between a nebulizer and an inhalation mask.

### BACKGROUND

Nebulizers are well known drug delivery means utilized in the medical industry in order to provide a drug entrained aerosol or mist from a drug containing solution or suspension, which drug entrained aerosol or mist being for inhalation by a patient in need thereof. The mist may for example include the drug in an aerosolized or an atomized form.

A typical nebulizer includes a reservoir for housing a drug entrained solution, a gas inlet into the reservoir to allow a gas (such as medical oxygen) to pass over or through the solution, an aerosol or mist forming means, typically a nozzle having a first orifice to receive drug entrained solution and gas and an opposite spray orifice from which the aerosol or mist is dispensed, and an outlet for receiving the aerosol or mist, which outlet extending away from the nozzle and reservoir and generally in flow communication with an inhalation mask or mouthpiece.

When in use, pressurized gas enters the nebulizer via the gas inlet, passing over or through the drug entrained solution before passing through the nozzle to produce a Venturi effect that facilitates drawing the drug entrained solution up and into the nozzle by means of a pressure differential such that said drug entrained solution is encouraged to form an aerosol or mist as it passes through the nozzle and/or when expelled through the spray orifice, through the outlet, and to the patient via the inhalation mask or mouthpiece. The nozzle typically provides a narrow passage and may taper towards its spray orifice.

A mechanically rudimentary nebulizer relying on only a tapering nozzle to facilitate aerosol or mist formation is known to be ineffective often resulting in non-aerosolized drug entrained droplets being administered to the patient. This situation is inimical since droplet size is important in administration regimes of certain drugs. Consequently, standard nebulizers have been adapted to provide a baffle arrangement (often shaped and/or dimensioned as an anvil arrangement) substantially adjacent the nozzle and spaced from the orifice to further and more effectively facilitate aerosol or mist formation and to hinder non-aerosolized droplets from being inhaled when in use.

In order to ensure proper functioning of the nebulizer when in use, the nozzle is required to remain vertical relative to the ground. This requires patients to be seated in a generally upright position, or to be standing, when inhaling drugs via a nebulizer creating for operational difficulties. Often patients are too weak to sit in the required position, are unconscious, are recovering from a general anaesthetic, or are under general anesthesia hindering placement of the patient in an appropriate position relative to the nozzle.

It is also common for the outlet to join the inhalation mask or mouthpiece via an elbowed joint, typically providing a bend of about 90°. The elbowed joint encourages the patient to sit in an upright position prior to using the nebulizer, however, it frustrates drug administration in situations where the patient cannot comply. Further, the elbowed joint provides a curved flow path and a surface upon which the aerosol or mist can condense providing non-aerosolized drug entrained droplets which may negatively impact on the efficacy of the drug delivery. It is also known that a longer length between the outlet and the inhalation mask or mouthpiece increases the risk of condensation.

US 2007/0049841 A1 describes a medication delivery apparatus and method for continuous positive airway pressure. The apparatus of the invention includes a 3-port tee fitting where one port is connected to the inlet port of a mask, the second port is connected to a gas conduit supplying oxygen, and the third port is connected to a flexible tube. The 3-port tee fitting provides a curved flow path and a surface upon which aerosol or mist can condense providing non-aerosolized drug entrained droplets which may negatively impact on the efficacy of drug delivery when employing a nebulizer.

US 2005/0092325 A1 describes a nebulizer connector tube comprising an elongated tube having a face mask connector collar and a nebulizer connector collar at opposite sides thereof, wherein the elongated tube is provided with a bellows type configuration along its length and is flexible and resilient. The bellows type configuration provides an irregular flow path and multiple surfaces upon which aerosol or mist can condense providing non-aerosolized drug entrained droplets which may negatively impact on the efficacy of drug delivery when employing a nebulizer.

The Spanish utility model ES 1 045 126 U teaches an adjustable device for medical use and is characterized by having a device that is sandwiched between a mask and an aerosol cup, wherein the device consists of two tubes bent at an angle of 90 degrees with a uniform diameter identical to that of the inlet of the mask and the outlet of the aerosol cup. The two bent tubes provide an irregular flow path and surfaces upon which aerosol or mist can condense providing non-aerosolized drug entrained droplets which may negatively impact on the efficacy of drug delivery when employing a nebulizer. US 2014/0166010 A1 discloses a nebulizer dispenser having a T-piece, a body sealingly coupled to the T-piece and configured to contain a liquid medication, and a breathing piece sealingly coupled to the T-piece. The dispenser also has a ball-and-socket joint coupled between the T-piece and either the breathing piece or the body. The T-piece provides an irregular flow path and surfaces upon which aerosol or mist can condense which is problematic.

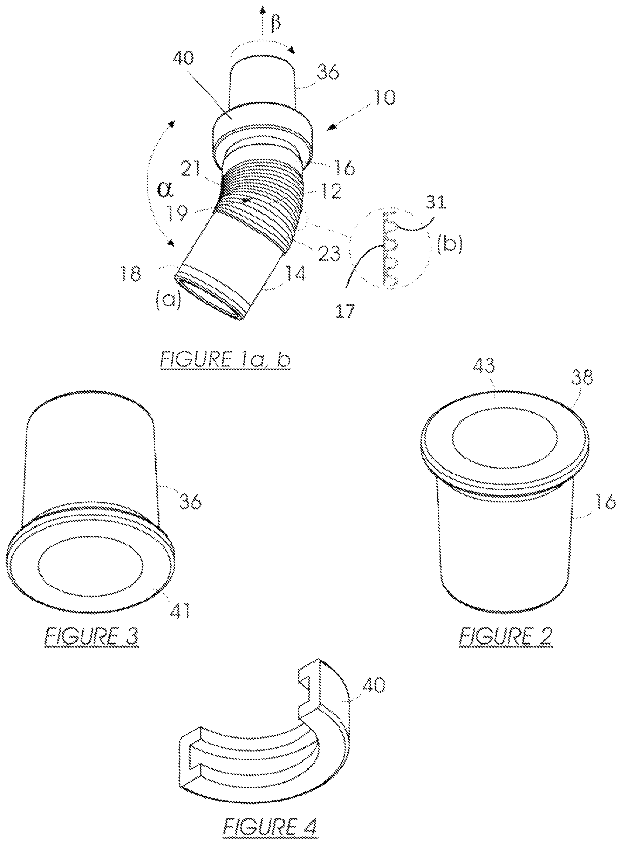

US 2017/0007797 A1 discloses a collapsible joint for use with a nebulizer and includes a housing having a top and bottom portion, a collapsible airway having two opposing ends, a first opposing end communicating with the top portion of the chamber defined in the housing, and an extension communicating with the second opposing end, and where the extension communicates the collapsible airway with at least a portion of the nebulizer for providing aerosolized medication to a patient. The housing is an essential feature of the nebulizer. The invention of US 2017/0007797 A1 cannot readily be retrofitted onto conventional nebulizers. The extension increases the flow path and a longer length between the outlet and the inhalation mask or mouthpiece increases the risk of condensation. The preferred embodiment depicted in Figure 4 shows two spaced ball and socket joints. Aerosolized drug from the nebulizer will flow onto and condense against the outer surface of the first ball reducing efficacy, and aerosolized drug that manages to in turn flow into the second ball will further condense against its inner surface before finally being expelled toward the patient for inhalation.

US 5,357,945 A discloses a nebulizer attachment comprising a reservoir chamber for liquid medication, a face attachment indirectly connected to said reservoir chamber, and a means for maintaining said reservoir chamber in a vertical position. The means for maintaining said reservoir chamber in a vertical position comprises a hollow fitting being formed of a plastic tube having a plurality of circumferential, unconnected corrugations of a semi-flexible nature. The corrugations provide an irregular flow path and surfaces upon which aerosol or mist can condense providing non-aerosolized drug entrained droplets which may negatively impact on the efficacy of drug delivery when employing a nebulizer.

US 2012/0285452 A1 discloses an adapter tube comprising a 90 degree elbow tube member. The 90 degree elbow encourages the patient to sit in an upright position prior to using a nebulizer, however, it frustrates drug administration in situations where the patient cannot comply.

There is a need to ameliorate at least one of the problems described above and/or known in the prior art.

### SUMMARY OF THE INVENTION

The present invention provides a nebulizer orientation apparatus as defined in claim 1. Further preferred embodiments of the present invention are defined in the dependent claims 2 to 9.

### SUMMARY OF THE PRESENT DISCLOSUR

Reference will now be made to various exemplary embodiments of the present disclosure. Embodiments disclosed herein which do not fall under the scope of the appended claims do not form part of the present invention, but are useful for understanding the principles of the invention. The scope of the present invention is defined by the appended claims.

Broadly, and in accordance with this general disclosure there is provided a nebulizer orientation apparatus for orientating an aerosol or mist forming means of the nebulizer into a substantially vertical position relative to the ground level under force of gravity.

In accordance with a first embodiment of this disclosure there is provided a nebulizer orientation apparatus for interposition between a nebulizer and an inhalation mask (or mouthpiece), the nebulizer orientation apparatus comprising:
first and second straight hollow bodies in flow communication with each other via a joint means, which joint means facilitating movement of the first and second straight hollow bodies relative to each other in a first plane defined (or facilitated )by the joint means, and
wherein the first straight hollow body is weighted relative to the second straight hollow body to facilitate displacement of the first body under force of gravity relative to the second body,
   such that in use,
   the nebulizer may be attached to the first body and the inhalation mask (or mouthpiece) may be attached to the second body, the nebulizer containing a drug entrained solution and the inhalation mask (or mouth piece) placed over a mouth and/or nose of a patient, and
wherein the first body together with the nebulizer may be displaced relative to the second body under force of gravity to ensure that the nebulizer, and particularly an aerosol or mist forming means of the nebulizer, is maintained substantially upright relative to ground level therein facilitating effective aerosol and/or mist formation when a torso and/or head of the patient is moved away from a substantially vertical position relative to the ground level.

The nebulizer orientation apparatus may further comprise a hollow connector rotationally mounted relative to an outlet portion of the second hollow body, wherein rotational movement of the hollow connector relative to the second hollow body is in a second plane. The second plane being substantially orthogonal to the first plane.

The joint means may comprise a deformable conduit.

The deformable conduit may comprise a metallic support embedded in a plastics material. The metallic support may be any one of a coil, helix, and/or lattice. The deformable conduit may be resilient. The deformable conduit may be expandable and/or contractable along its length. The deformable conduit may be provided with corrugations along its length to facilitate expansion. The deformable conduit may have a smooth interior bore.

The deformable conduit may include a mid-portion having a larger diameter relative to opposing end portions.

The hollow connector may be rotationally mounted relative to the outlet portion of the second hollow body via an attachment means, wherein the attachment means facilitating rotational movement of the hollow connector relative to the second hollow body in the second plane substantially orthogonal to the first plane.

The attachment means may be a sleeve securing the outlet portion of the second hollow body to the hollow connector whilst concomitantly providing rotational motion relative to each other.

The hollow connector may matingly engage the outlet of the second hollow body whilst being adapted to concomitantly provide for rotational motion of the second hollow body relative to the hollow connector.

The first and second straight hollow bodies may each matingly engage the joint means. Alternatively, or additionally, the first and second straight hollow bodies may be integrally formed with the joint means.

The first and second straight hollow bodies may be formed from a plastics material.

The first hollow body may be weighted by being formed from a heavier material relative to the second hollow body, therein facilitating displacement of the first hollow body under force of gravity relative to the second hollow body.

The first and second hollow bodies may be manufactured from substantially the same material, and the first hollow body may weighted by being greater in length relative to the second hollow body, so as to provide the first hollow body with a greater mass relative to the second hollow body, therein facilitating displacement of the first hollow body under force of gravity relative to the second hollow body.

The first hollow body may be weighted by having attached thereto a weight means.

The weight means may be a collar and/or a ring at least partially surrounding the first hollow body. The weight means may be integrally formed with the first hollow body.

In a particular example of the first embodiment of this disclosure the weight means may be a thickening of a portion of a sidewall providing the first hollow body.

The weight means may be manufactured from a metallic material.

In accordance with a second embodiment (not claimed) of this disclosure there is provided a nebulizer orientation apparatus for interposition between a nebulizer and an inhalation mask (or mouthpiece), the nebulizer orientation apparatus comprising:
first and second straight hollow bodies in flow communication with each other via a hinged joint, wherein the first straight hollow body is weighted relative to the second straight hollow body to facilitate displacement of the first body under force of gravity relative to the second body; and
a hollow connector rotationally secured against an outlet of the second hollow body in flow communication with the inhalation mask (or mouthpiece), the hollow connector allowing rotational movement via an attachment means relative to the second hollow body, hinge and first hollow body,
such that in use,
the weight of the first hollow body facilitates displacement of the first hollow body under force of gravity relative to the second hollow body, said displacement being in a first plane defined (or facilitated) by the hinged joint, and
wherein the attachment means facilitating rotational movement of the hollow connector relative to the second hollow body in a second plane. The second plane being substantially orthogonal to the first plane.

The attachment means may be a sleeve securing an outlet portion of the second hollow body to the hollow connector whilst concomitantly providing rotational motion relative to each other.

The hinged joint may include a first pair of lugs attached to the first hollow body, and a second corresponding pair of lugs attached to the second hollow body, wherein the first and second pair of lugs are secured via nuts and bolts, alternatively, secured by way of interlocking studs and corresponding apertures.

The hinged joint may further include thereabout, and spanning the first and second hollow bodies, a closure in order to provide a closed flow path from the first hollow body, through the hinged joint, through the second hollow body, into and through the hollow connector and into the inhalation means (or mouthpiece) to be inhaled by the patient.

The closure may be a resiliently deformable closure to allow hinged movement of the first and second hollow bodies relative to each other through a section of the first plane.

The first and second straight hollow bodies may be formed from a plastics material.

The first hollow body may be weighted by being formed from a heavier material relative to the second hollow body, therein facilitating displacement of the first hollow body under force of gravity relative to the second hollow body.

The first and second hollow bodies may be manufactured from substantially the same material, and the first hollow body may weighted by being greater in length relative to the second hollow body, so as to provide the first hollow body with a greater mass relative to the second hollow body, therein facilitating displacement of the first hollow body under force of gravity relative to the second hollow body.

The first hollow body may be weighted by having attached thereto a weight means.

The weight means may be a collar and/or a ring at least partially surrounding the first hollow body. The weight means may be integrally formed with the first hollow body.

In a particular example of the second embodiment of this disclosure the weight means may be a thickening of a portion of a sidewall providing the first hollow body.

The weight means may be manufactured from a metallic material.

In accordance with a third embodiment (not claimed) of this disclosure there is provided a nebulizer orientation apparatus for interposition between a nebulizer and an inhalation mask (or mouthpiece), the nebulizer orientation apparatus comprising:
a swivel joint having extending from opposite ends thereof first and second straight hollow bodies, respectively, wherein the first straight hollow body is weighted relative to the second straight hollow body,
such that in use,
the weight of the first straight hollow body facilitates displacement of the first straight hollow body under force of gravity relative to the second straight hollow body.

The swivel joint may comprise a ball and socket arrangement, wherein the socket defines therethrough an aperture and is provided as a female frusto-spherical member, and wherein the ball may be provided as a complementarily shaped and dimensioned male frusto-spherical member defining therein a cavity such that the male and female members matingly engage to provide a flow path from the first hollow body through the swivel joint and out through the second hollow body.

The swivel joint may facilitate swiveled displacement and/or pivotal displacement of the first and second hollow bodies thereabout.

In a particular example of the third embodiment of this disclosure the female frusto-spherical member of the swivel joint is attached to the first hollow body, and the complementarily shaped and dimensioned male frusto-spherical member of the swivel joint is attached to the second hollow body. It is to be understood that the female frusto-spherical member of the swivel joint may be attached to the second hollow body, and the complementarily shaped and dimensioned male frusto-spherical member of the swivel joint may be attached to the first hollow body without departing from the scope of this disclosure.

The female frusto-spherical member and/or the complimentarily shaped and dimensioned male frusto-spherical member may be formed from a resilient material to facilitate mating engagement.

The first and second straight hollow bodies may be formed from a plastics material.

The first hollow body may be weighted by being formed from a heavier material relative to the second hollow body, therein facilitating displacement of the first hollow body under force of gravity relative to the second hollow body.

The first and second hollow bodies may be manufactured from substantially the same material, and the first hollow body may weighted by being greater in length relative to the second hollow body, so as to provide the first hollow body with a greater mass relative to the second hollow body, therein facilitating displacement of the first hollow body under force of gravity relative to the second hollow body.

The first hollow body may be weighted by having attached thereto a weight means.

The weight means may be a collar and/or a ring at least partially surrounding the first hollow body. The weight means may be integrally formed with the first hollow body.

In a particular example of the third embodiment of this disclosure the weight means may be a thickening of a portion of a sidewall providing the first hollow body.

The weight means may be manufactured from a metallic material.

The swivel joint may include a securing means to ensure that the male frusto-spherical member remains matingly engaged inside the female frusto-spherical member whilst still facilitating swiveled displacement and/or pivotal displacement relative to each other.

The securing means may include a first flange extending radially outwardly away from a periphery which defines an open mouth of the female frusto-spherical member, and a second corresponding flange for securement to the first flange, the second corresponding flange having depending downwardly away therefrom a frusto-spherical side wall, the second flange being locatable over the second body to secure against the first flange, wherein the frusto-spherical sidewall prevents disengagement of the frusto-spherical male member from the female frusto-spherical member.

The first and second flange may be joined in securing abutment through various adhesive means, for example, glue. In a certain embodiment, the first and second flanges may be spaced by a rubber ring.

There is provided for the swivel joint to provide a seal in order to ensure all aerosol or mist travels through the flow path toward the inhalation mask (or mouthpiece). In certain embodiments the swivel joint does not to provide a seal in order to substantially equalize pressure relative to atmospheric pressure. For example, the swivel joint may be provided with a hole through a wall thereof to ensure equalization of pressure to atmospheric pressure.

It is to be understood that the shape and dimension of the swivel joint may extend to include, but not limitingly so, discoid, geoid, oblong and elliptical type shapes and dimensions.

In accordance with a fourth embodiment (not claimed) of this disclosure there is provided a method of nebulizing a patient utilizing the nebulizer orientation apparatus described any one of the first, second or third embodiments of the disclosure herein, the method comprising the following steps:
connecting a nebulizer containing drug entrained solution therein to the first hollow body;
connecting the inhalation mask (or mouthpiece) to the second hollow body, preferably via a tube;
placing the inhalation mask (or mouthpiece) about an oral and/or nasal cavity of the patient;
actuating the nebulizer, in so doing,
allowing the drug entrained solution to aerosolize and/or atomize into a drug entrained aerosol or mist through an aerosol or mist forming means, the drug entrained aerosol or mist flowing through the joint means into and through the second hollow body, through the inhalation means (or mouthpiece) and into the oral and/or nasal cavity of the patient.

The aerosol or mist forming means may be a nozzle. Preferably, the nozzle may taper from a first orifice which in use receives drug entrained solution and gas to an opposite spray orifice from which the aerosol or mist is dispensed.

The weighted first hollow body facilitates maintenance of the nozzle in a substantially vertical position relative to the ground so as to ensure proper aerosol and/or mist formation.

The straight first and second hollow bodies provide a near straight flow path hindering condensation, and facilitating appropriate droplet size and/or appropriate drug dosage regimes.

### BRIEF DESCRIPTION OF THE DRAWINGS

Embodiments of the invention will be described below by way of example only and with reference to the accompanying diagrammatic drawings in which:
- **FIGURE 1a**: shows a three dimensional view of a nebulizer orientation apparatus according to a first embodiment of this disclosure, including first and second hollow bodies and a joint means there between, and further including a connector and attachment means for rotational mounting of the connector to the second hollow body;
- **FIGURE 1b**: shows a more detailed view of the joint means of Figure 1 including a smooth inner bore and outer corrugations;
- **FIGURE 2**: shows the second hollow body of Figure 1 defining an outlet portion and a second near flat collar;
- **FIGURE 3**: shows the connector of Figure 1 having a first near flat collar for rotational mounting to the second near flat collar of the second hollow body shown in Figure 2;
- **FIGURE 4**: shows a portion through the attachment means, which is provided as a sleeve, of Figure 1 for rotatably joining the second hollow body to the connector in the first embodiment of this disclosure;
- **FIGURE 5**: shows a three dimensional view of the nebulizer orientation apparatus according to a second embodiment (not claimed) of this disclosure including a hinged joint having extending therefrom first and second hollow bodies;
- **FIGURE 6**: shows the first hollow body of Figure 5 having a first pair of lugs to form the hinged joint;
- **FIGURE 7**: shows the second hollow body of Figure 5 having a second corresponding pair of lugs to form the hinged joint;
- **FIGURE 8**: shows a hollow connector of the nebulizer orientation apparatus of Figure 5;
- **FIGURE 9**: shows portion through the attachment means (which is provided as a sleeve) for rotatably joining the second hollow body to the connector in the second embodiment of this disclosure;
- **FIGURE 10**: shows a three dimensional view of a nebulizer orientation apparatus according to a third embodiment (not claimed) of this disclosure including a swivel joint having extending from opposite ends thereof first and second straight hollow bodies;
- **FIGURE 11**: shows the first straight hollow body and a male frusto-spherical member of swivel joint shown in Figure 10;
- **FIGURE 12**: shows the second straight hollow body and a female frusto-spherical member of the swivel joint including a first flange of the securing means, as shown in Figure 10;
- **FIGURE 13**: shows a second corresponding flange of the securing means including a frustospherical side wall to ensure mating engagement of the male and female members of the swivel j oint;
- **FIGURE 14**: shows the first embodiment of the disclosure is use by a patient;
- **FIGURE 15**: shows the second embodiment of the disclosure in use by a patient; and
- **FIGURE 16**: shows the third embodiment of the disclosure in use by a patient.

### DETAILED DESCRIPTION

The Summary of the disclosure is repeated hereunder by way of reference thereto only to avoid repetition. Like structural elements in the different embodiments are provided with like or corresponding reference numerals in as far as is possible.

Generally, and in accordance with a first embodiment of this disclosure there is provided a nebulizer orientation apparatus 10 for interposition between a nebulizer 302 and an inhalation mask (or mouthpiece) 304 (see Figures 1 to 4 and 14). The nebulizer orientation apparatus 10 comprises first and second straight hollow bodies 14, 16 in flow communication with each other via a joint means 12. The joint means 12 facilitates movement of the first and second straight hollow bodies 14,16 relative to each other in a first plane (α) defined by the joint means 12.

Typically, the first and second straight hollow bodies 14, 16 each matingly engage the joint means 12.

According to the present invention, the first and second straight hollow bodies 14, 16 and the joint means are integrally formed.

The first straight hollow body 14 is weighted (using a strap 18) relative to the second straight hollow body 16 to facilitate displacement of the first body 14 under force of gravity relative to the second body 16. This is shown in Figure 1. Other weight means are envisaged by the Applicant.

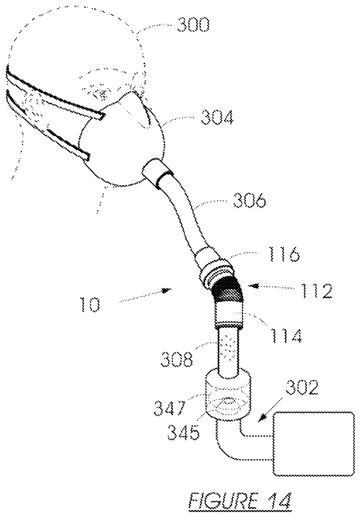

In use, the nebulizer 302 is attached to the first body 14 and the inhalation mask (or mouthpiece) 304 is attached to the second body 16, as shown in Figure 14. The nebulizer 302 (shown diagrammatically) contains a drug entrained solution 347 and the inhalation mask (or mouth piece) 304 placed over a mouth and/or nose of a patient 300, wherein the first body 14 together with the nebulizer may be displaced relative to the second body 16 under force of gravity to ensure that the nebulizer 302, and particularly an aerosol or mist forming means 345 of the nebulizer, is maintained substantially upright relative to ground level therein facilitating effective aerosol and/or mist formation when a torso and/or head of the patient 300 is moved away from a substantially vertical position relative to the ground level. The nebulizer orientation apparatus 10 further comprises a hollow connector 36 rotationally mounted relative to an outlet portion 38 of the second hollow body 16. The hollow connector is shown in Figure 3. The rotational movement of the hollow connector 36 relative to the second hollow body 16 is in a second plane (β). The second plane (β) is substantially orthogonal to the first plane (α). As such, this first embodiment of this disclosure provides for at least two degrees of freedom (the first via the joint means 12, and the second between the hollow connector 36 and the second hollow body 16) which is beneficial and enhances patient compliance. In use, the nebulizer may be displaced to remain substantially upright relative to the ground via movement in the plane (α) and the plane (β).

The joint means 12 is typically provided as a deformable conduit. The deformable conduit may comprise a metallic support embedded in a plastics material. The metallic support may be any one of a coil, helix, and/or lattice. The deformable conduit may be resilient. Typically, medical grade plastics material is used, and optionally further embedding a metallic support.

In a certain embodiment of this disclosure, the deformable conduit includes a mid-portion 19 and opposing end portions 21,23. The mid-portion 19 may have a larger diameter relative to the opposing end portions 21, 23 (not shown). The mid-portion 19 of the joint means 12 is typically provided with corrugations 31 on an outer surface thereof to facilitate selective expansion and/or contraction along the length of the mid-portion 19. An inner bore 17 of the joint means 12 is smooth. The smooth inner bore 17 improves flow of drug entrained aerosol when in use.

The hollow connector 36 is rotationally mounted relative to the outlet portion 38 of the second hollow body 16 via an attachment means 40, wherein the attachment means 40 facilitates rotational movement of the hollow connector 36 relative to the second hollow body 16 in a second plane (β) substantially orthogonal to the first plane (α).

The Applicant envisages other forms of rotational mounting. For example, the hollow connector 36 may matingly engage the second hollow body 16 whilst concomitantly providing for rotational motion relative to each other in the plane shown in the figures as (β).

The hollow connector 36 defines a first near flat collar 41 which abuts a corresponding or similar second near flat collar 43 defined by the outlet portion 38 of the second hollow body 16. This is shown in Figures 2 and 3. It is envisaged that the two near flat collars 41, 43 may include mating means to allow for fitting engagement with each other, for example, but not limited to a protruding circular ridge to engage a corresponding circular trough (not shown). The attachments means in Figure 1 and 4 is provided for as a sleeve 40 securing the outlet portion 38 of the second hollow body 16 to the hollow connector 36 whilst concomitantly providing rotational motion relative to each other. The rotational motion is shown in the figures as plane (β). A half cut through portion of the sleeve 40 is shown in Figure 4. Similar attachment means 40 to clamp together the second hollow body 16 and the connector 36 whilst providing rotational motion in plane (β) are envisaged.

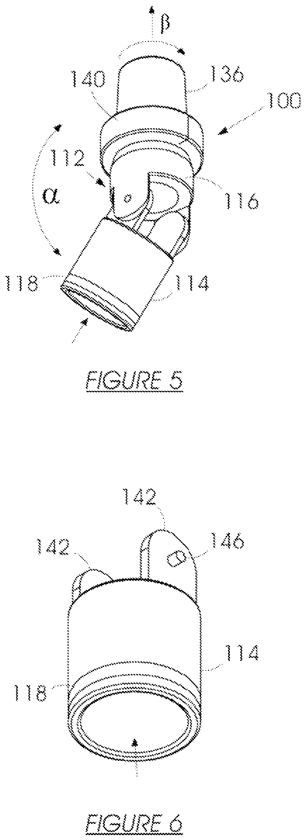

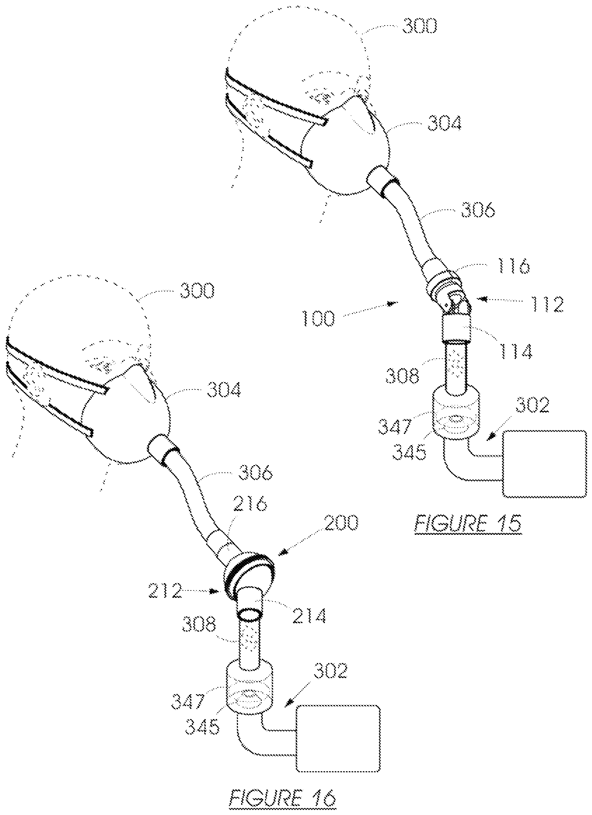

Generally, and in accordance with a second embodiment of this disclosure there is provided a nebulizer orientation apparatus 100 for interposition between a nebulizer and an inhalation mask (or mouthpiece). The second embodiment (not claimed) is shown in Figures 5 to 9 and in use in Figure 15. The nebulizer orientation apparatus 100 comprises first and second straight hollow bodies 114, 116 in flow communication with each other via a hinged joint 112. The first straight hollow body 114 is weighted relative to the second straight hollow body 116.

The additional weight of the first straight hollow body 114 relative to the second 116 is provided by a weight means shown in Figure 6 as a strap 118. The strap 118 facilitates displacement of the first body under force of gravity relative to the second body.

The apparatus 100 further comprises a hollow connector 136 rotationally secured against an outlet portion 138 of the second hollow body 116 and in flow communication with the inhalation mask (or mouthpiece). The flow is illustrated by headed arrows. The hollow connector 136 allows rotational movement via an attachment means 140 relative to all of the second hollow body 116, the hinge 112 and first hollow body 114.

When in use, the strap 118 of the first hollow body 114 facilitates displacement of the first hollow body 114 under force of gravity relative to the second hollow body 116, said displacement being in a first plane (α) defined by the hinged joint 112.

The attachment means 140 facilitates rotational movement of the hollow connector 136 relative to all of the second hollow body 116, the hinge 112 and the first hollow body 114, in a second plane (β). The second plane (β) is substantially orthogonal to the first plane (α).

The attachment means 140 may be a sleeve securing the outlet 138 of the second hollow body 116 to the hollow connector 136 whilst concomitantly providing rotational motion (β) relative to each other.

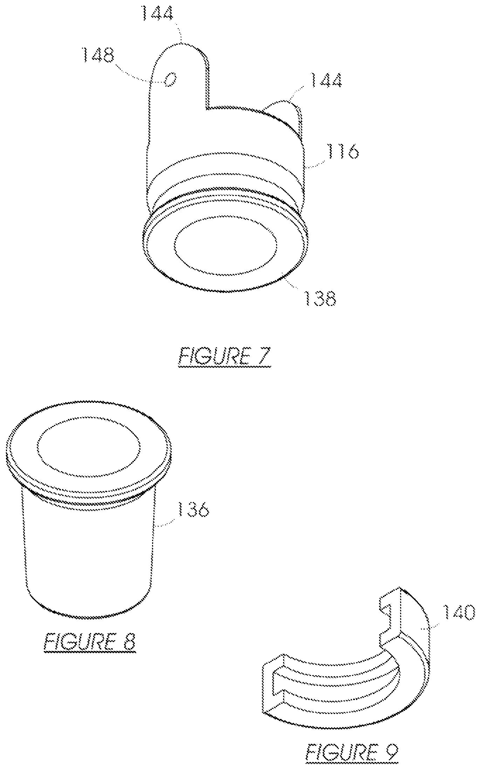

Figure 9 shows half of the attachment means being in the form of a sleeve.

The hinged j oint 112 may include may include a first pair of lugs 142 attached to the first hollow body 114, and a second corresponding pair of lugs 144 attached to the second hollow body 116, wherein the first and second pair of lugs 142, 144 are secured via interlocking stud 146 and aperture 148 means, respectively. It is to be understood that the lugs 142, 144 may be hingingly secured via alternative means, such as for example, nuts and bolts (not shown).

The hinged joint 112 may further include thereabout, and spanning the first and second hollow bodies 114, 116, a closure (not shown) in order to provide a closed flow path from the first hollow body 114, through the hinged joint 112, through the second hollow body 116, into and through the hollow connector 136 and into the inhalation means (or mouthpiece) to be inhaled by the patient.

The closure may be a resiliently deformable closure to allow hinged movement of the first and second hollow bodies 114, 116 relative to each other through a section of the first plane (α). The closure may be an airtight balloon means surrounding the joint means 112 providing a closed flow path.

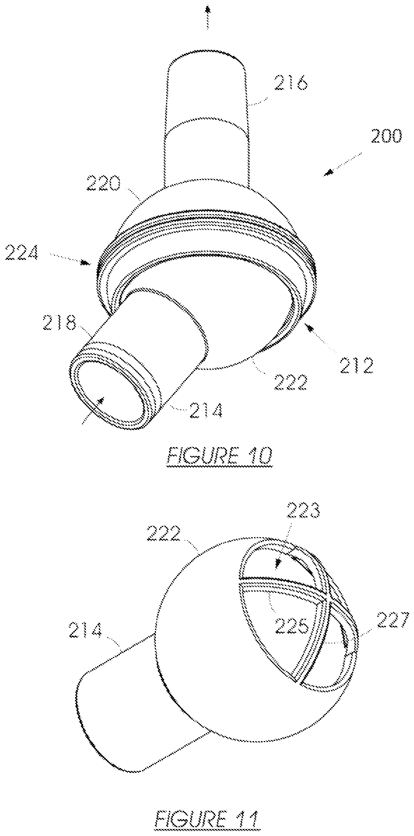

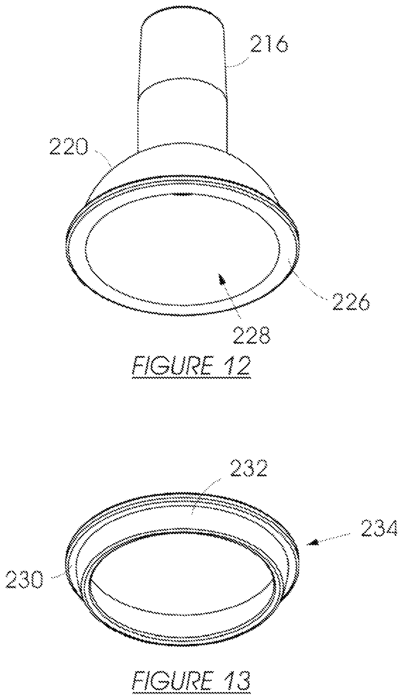

Generally, and in accordance with a third embodiment (not claimed) of this disclosure there is provided a nebulizer orientation apparatus 200 illustrated in Figures 10 to 13 and Figure 16. The third embodiment of the nebulizer orientation apparatus 200 is shown in use on a patient in Figure 15.

In accordance with a third embodiment of this disclosure there is provided a nebulizer orientation apparatus 200. The apparatus 200 is for interposition between a nebulizer and an inhalation mask (or mouthpiece) (as shown in Figure 16) when providing a drug to a patient via inhalation.

The apparatus 200 comprises a swivel joint 212 having extending from opposite ends thereof first and second straight hollow bodies 214, 216, respectively. The first straight hollow body 214 is weighted relative to the second straight hollow body 216 by attaching to the first hollow body a weight means in the forms of a strap 218.

In use, the weight of the first hollow body 214 facilitates displacement thereof under force of gravity relative to the second hollow body 216.

The swivel joint 212 is shown to comprise a ball and socket arrangement, wherein the socket defines therethrough an aperture and is provided as a female frusto-spherical member 220, and wherein the ball may be provided as a complementarily shaped and dimensioned male frusto-spherical member 222 defining therein a cavity 223 such that the male and female members matingly engage to provide a flow path (indicated by headed arrows) from the first hollow body 214 through the swivel joint 212 and out through the second hollow body 216. In Figure 11, the cavity 223 is spanned by two arches 225, 227 crossing at a midway point. It is to be understood that the arches 225, 227 are illustrative only, and do not form an essential component of the apparatus 200.

In the illustrated example embodiments of Figures 10 to 13, the female frusto-spherical member 220 of the swivel joint 212 is attached to the second hollow body 216, and the complementarily shaped and dimensioned male frusto-spherical member 222 of the swivel joint 212 is attached to the first hollow body 214. It is to be understood that the female frusto-spherical member 220 of the swivel joint 212 may alternatively be attached to the first hollow body 214, and the complementarily shaped and dimensioned male frusto-spherical member 222 of the swivel joint 212 may be attached to the second hollow body 216 without departing from the scope of this disclosure.

In Figures 10 to 13, the first hollow body 214 is weighted by providing there around a strap 218. It is to be understood that the additional weight of the first hollow member 214 may be provided by manufacturing the first hollow member 214 from a heavier material relative to the second hollow body 216. This is not illustrated.

Alternatively, the first and second hollow bodies 214, 216 may be manufactured from substantially the same material, and the first hollow body 214 may weighted by being greater in length relative to the second hollow body 216, so as to provide the first hollow body 214 with a greater mass relative to the second hollow body 216. This is not illustrated.

It is further to be understood that the weight means may be integrally formed with the first hollow body 214. In a particular example of the third embodiment of this disclosure the weight means may be a thickening of a portion of a sidewall providing the first hollow body 214 (not shown).

The swivel joint 212 is shown to include a securing means 224 to ensure that the male frusto-spherical member 222 remains matingly engaged inside the female frusto-spherical member 220 whilst still facilitating swiveled displacement and/or pivotal displacement relative to each other.

The securing means 224 comprises a first flange 226 extending radially outwardly away from a periphery which defines an open mouth 228 of the female frusto-spherical member 220. The securing means 224 further comprises a second corresponding flange 230 for securement to the first flange 226, the second corresponding flange 230 having depending downwardly away therefrom a frusto-spherical side wall 232. The second flange 230 and sidewall 232 are together provided as a ring 234. The first and second flanges 226, 230 have oppositely located flat near circular faces to allow for operative abutment in use.

The ring 234 is locatable over the first body 214 when the male member 222 is matingly engaged with the female member 220 to secure the second flange 230 against the first flange 226 such that the frusto-spherical sidewall 232 prevents disengagement of the frusto-spherical male member 222 from the female frusto-spherical member 220.

The first and second flange 226, 230, may be joined in securing abutment through various means, for example, glue. In a certain embodiment, the first and second flanges 226, 230 may be spaced by a rubber ring.

It is to be understood that the swivel joint 212 may provide a seal in order to ensure all aerosol or mist travels through the flow path toward the inhalation mask (or mouthpiece) without escaping through the swivel joint 212. In certain embodiments the swivel joint 212 does not to provide a seal in order to substantially equalize pressure relative to atmospheric pressure.

The third embodiment of the nebulizer orientation apparatus 200 is shown in use on a patient 300 in Figure 16.

In Figures 14-16 the nebulizer orientation apparatus 10, 100, 200 is located between a nebulizer 302 and an inhalation mask (or mouthpiece) 304. The inhalation mask (or mouthpiece) 304 is attached to the apparatus 10, 100, 200 via a substantially flexible tube 306 typically manufactured from a medical grade plastics material. The nebulizer 302 is attached to the apparatus 10, 100, 200 via a connector means 308. The connector means 308 means may be flexible. The flexible tube 306 and/or the connector means 308 may each or both be provided as plastics tubing being reinforced with a metallic coil, or a series of metallic rings. The coil and/or rings may be embedded within a plastics material forming the tubing. Additionally, or alternatively, the flexible tube and/or connector means 308 may each or both be provided as expandable tubing. Typically, expandable tubing is provided with corrugations along its length.

The first and second straight hollow bodies facilitate ensuring that drug entrained aerosol or mist remains in an aerosolized and/or atomized form when in use.

In accordance with a fourth embodiment (not claimed) of this disclosure there is provided a method of nebulizing a patient utilizing the nebulizer orientation apparatus 10, 100, 200, described in the first, second and/or third embodiments of the disclosure herein above. The method is not specifically illustrated but is exemplified and/or described here below.

The method comprises the following steps:
connecting a nebulizer 302 containing drug entrained solution 347 therein to the first hollow body 14, 114, 214;
connecting the inhalation mask (or mouthpiece) 304 to the second hollow body 16, 116, 216, preferably via a first tube 306;
placing the inhalation mask (or mouthpiece) 304 about an oral and/or nasal cavity of the patient 300;
actuating the nebulizer 302, in so doing,
allowing the drug entrained solution 347 to aerosolize and/or atomize into a drug entrained aerosol or mist through an aerosol or mist forming means 345.

The drug entrained aerosol or mist travels through the flow path (indicated by headed arrows in the figures) flowing through the joint means 12, 112, 212 into and through the second hollow body 16, 116, 216, through the inhalation means (or mouthpiece) 304 and into the oral and/or nasal cavity of the patient 300.

The aerosol or mist forming means 345 is typically a nozzle found in standard nebulizers. Preferably, the nozzle may taper from a first orifice which in use receives drug entrained solution and gas to an opposite spray orifice from which the aerosol or mist is dispensed (this is not shown in detail in the figures).

The Applicant has found that the first and second straight hollow bodies 14, 16 (114, 116) (214, 216) provide a flow path through the apparatus 10, 100, 200 that is substantially unobstructed hindering condensation of aerosol or mist, and that the weighted first straight hollow member 14, 114, 214 enables the aerosol or mist forming means (typically the nozzle) of the nebulizer 302 to remain substantially vertical relative to the ground, and in so doing, ensuring proper nebulization or aerosol or mist formation from the drug entrained solution 347.

While the disclosure has been described in detail with respect to specific embodiments and/or examples thereof, it will be appreciated that those skilled in the art, upon attaining an understanding of the foregoing may readily conceive of alterations to, variations of and equivalents to these embodiments.

The scope of the present invention is defined by the following appended claims.

## Claims

1. A nebulizer orientation apparatus (10) for interposition between a nebulizer (302) and an inhalation mask (304), the nebulizer orientation apparatus comprising first and second straight hollow bodies (14, 16) in flow communication with each other via a joint means (12), wherein the joint means is a deformable conduit including a smooth inner bore (17) and is expandable and/or contractable along its length, which joint means facilitating movement of the first and second straight hollow bodies relative to each other in a first plane (α) defined by the joint means, and wherein the first straight hollow body is weighted relative to the second straight hollow body to facilitate displacement of the first body under force of gravity relative to the second body, and wherein the first and second straight hollow bodies are integrally formed with the joint means.

2. The nebulizer orientation apparatus of Claim 1, wherein the deformable conduit comprises a metallic support embedded in a plastics material, wherein the metallic support is any one selected from the group: coil, helix and lattice, and wherein the deformable conduit is resilient, and wherein the deformable conduit is provided with corrugations (31) to facilitate expansion and/or contraction along its length, and wherein the deformable conduit includes a mid-portion (19) having a larger diameter relative to opposing end portions (21, 23).

3. The nebulizer orientation apparatus of any one of Claims 1 to 2, further comprising a hollow connector (36) rotationally mounted relative to an outlet portion (38) of the second hollow body (16), wherein rotational movement of the hollow connector relative to the second hollow body is in a second plane (β) substantially orthogonal to the first plane (α), wherein the hollow connector is rotationally mounted relative to the outlet portion of the second hollow body via an attachment means (40), and wherein the attachment means facilitates rotational movement of the hollow connector relative to the second hollow body in the second plane, and wherein the attachment means is provided as a sleeve securing the outlet portion of the second hollow body to the hollow connector whilst concomitantly providing rotational motion relative to each other, and wherein the hollow connector matingly engages the outlet of the second hollow body whilst being adapted to concomitantly provide for rotational motion of the second hollow body relative to the hollow connector.

4. The nebulizer orientation apparatus of any one of Claims 1 to 3, wherein the first hollow body (14) is weighted by being formed from a heavier material relative to the second hollow body (16), therein facilitating displacement of the first hollow body under force of gravity relative to the second hollow body.

5. The nebulizer orientation apparatus of any one of Claims 1 to 3, wherein, the first and second hollow bodies are manufactured from the same material, and the first hollow body is weighted by being greater in length relative to the second hollow body, so as to provide the first hollow body with a greater mass relative to the second hollow body, therein facilitating displacement of the first hollow body under force of gravity relative to the second hollow body.

6. The nebulizer orientation apparatus of any one of Claims 1 to 3, wherein, the first hollow body is weighted by having attached thereto a weight means.

7. The nebulizer orientation apparatus of Claim 6, wherein the weight means is a collar and/or a ring at least partially surrounding the first hollow body.

8. The nebulizer orientation apparatus of Claim 7, wherein the weight means is integrally formed with the first hollow body.

9. The nebulizer orientation apparatus of Claim 8, wherein the weight means is a thickening of a portion of a sidewall providing the first hollow body.

## Patentansprüche

1. Vernebler-Ausrichtungsvorrichtung (10) zur Zwischenschaltung zwischen einen Vernebler (302) und eine Inhalationsmaske (304), wobei die Vernebler-Ausrichtungsvorrichtung einen ersten und einen zweiten geraden Hohlkörper (14, 16) umfasst, die über ein Verbindungsmittel (12) in Strömungsverbindung miteinander stehen, wobei das Verbindungsmittel eine verformbare Leitung mit einer glatten Innenbohrung (17) ist und entlang seiner Länge ausziehbar und/oder zusammenschiebbar ist, wobei das Verbindungsmittel die Bewegung des ersten und des zweiten geraden Hohlkörpers relativ zueinander in einer ersten Ebene (α) erleichtert, die durch das Verbindungsmittel definiert ist, und wobei der erste gerade Hohlkörper relativ zu dem zweiten geraden Hohlkörper beschwert ist, um die Verlagerung des ersten Körpers unter Schwerkraft relativ zu dem zweiten Körper zu erleichtern, und wobei der erste und der zweite gerade Hohlkörper einstückig mit dem Verbindungsmittel ausgebildet sind.

2. Vernebler-Ausrichtungsvorrichtung nach Anspruch 1, wobei die verformbare Leitung einen metallischen Träger umfasst, der in ein Kunststoffmaterial eingebettet ist, wobei der metallische Träger einer ist, der ausgewählt ist aus der Gruppe: Spule, Spirale und Gitter, und wobei die verformbare Leitung elastisch ist, und wobei die verformbare Leitung mit Riffelungen (31) versehen ist, um die Ausdehnung und/oder Kontraktion entlang ihrer Länge zu erleichtern, und wobei die verformbare Leitung einen Mittelabschnitt (19) mit einem größeren Durchmesser relativ zu gegenüberliegenden Endabschnitten (21, 23) umfasst.

3. Vernebler-Ausrichtungsvorrichtung nach einem der Ansprüche 1 bis 2, die ferner ein hohles Verbindungsstück (36) umfasst, das relativ zu einem Auslassabschnitt (38) des zweiten Hohlkörpers (16) drehbar angebracht ist, wobei eine Drehbewegung des hohlen Verbindungsstücks relativ zu dem zweiten Hohlkörper in einer zweiten Ebene (β) erfolgt, die im Wesentlichen orthogonal zu der ersten Ebene (α) ist, wobei das hohle Verbindungsstück relativ zu dem Auslassabschnitt des zweiten Hohlkörpers über ein Befestigungsmittel (40) drehbar angebracht ist, und wobei das Befestigungsmittel eine Drehbewegung des hohlen Verbinders relativ zu dem zweiten Hohlkörper in der zweiten Ebene erleichtert, und wobei das Befestigungsmittel als eine Hülse vorgesehen ist, die den Auslassabschnitt des zweiten Hohlkörpers an dem hohlen Verbinder sichert, während sie gleichzeitig eine Drehbewegung relativ zueinander ermöglicht, und wobei der hohle Verbinder passend in den Auslass des zweiten Hohlkörpers eingreift, während er angepasst ist, um gleichzeitig eine Drehbewegung des zweiten Hohlkörpers relativ zu dem hohlen Verbinder zu ermöglichen.

4. Vernebler-Ausrichtungsvorrichtung nach einem der Ansprüche 1 bis 3, wobei der erste Hohlkörper (14) dadurch beschwert ist, dass er aus einem schwereren Material als der zweite Hohlkörper (16) gebildet ist, wodurch die Verlagerung des ersten Hohlkörpers unter Schwerkraft relativ zum zweiten Hohlkörper erleichtert wird.

5. Vernebler-Ausrichtungsvorrichtung nach einem der Ansprüche 1 bis 3, wobei der erste und der zweite Hohlkörper aus demselben Material hergestellt sind und der erste Hohlkörper durch eine größere Länge im Verhältnis zum zweiten Hohlkörper beschwert ist, um den ersten Hohlkörper mit einer größeren Masse im Verhältnis zum zweiten Hohlkörper zu versehen, wodurch die Verlagerung des ersten Hohlkörpers unter Schwerkraft relativ zum zweiten Hohlkörper erleichtert wird.

6. Vernebler-Ausrichtungsvorrichtung nach einem der Ansprüche 1 bis 3, wobei der erste Hohlkörper durch ein daran befestigtes Gewichtsmittel beschwert ist.

7. Vernebler-Ausrichtungsvorrichtung nach Anspruch 6, wobei das Gewichtsmittel ein Kragen und/oder ein Ring ist, der den ersten Hohlkörper zumindest teilweise umgibt.

8. Vernebler-Ausrichtungsvorrichtung nach Anspruch 7, wobei das Gewichtsmittel einstückig mit dem ersten Hohlkörper ausgebildet ist.

9. Vernebler-Ausrichtungsvorrichtung nach Anspruch 8, wobei das Gewichtsmittel eine Verdickung eines Teils einer Seitenwand ist, die den ersten Hohlkörper bildet.

## Revendications

1. Appareil d'orientation de nébuliseur (10) destiné à être intercalé entre un nébuliseur (302) et un masque d'inhalation (304), l'appareil d'orientation de nébuliseur comprenant des premier et second corps creux droits (14, 16) en communication d'écoulement entre eux via un moyen de joint (12), dans lequel le moyen de joint est un conduit déformable comprenant un alésage interne lisse (17) et est expansible et/ou contractile le long de sa longueur, lequel moyen de joint facilitant le mouvement des premier et second corps creux droits l'un par rapport à l'autre dans un premier plan (α) défini par le moyen de joint, et dans lequel le premier corps creux droit est pondéré par rapport au second corps creux droit afin de faciliter le déplacement du premier corps sous une force de gravité par rapport au second corps, et dans lequel les premier et second corps creux droits sont formés, de manière solidaire, avec le moyen de joint.

2. Appareil d'orientation de nébuliseur selon la revendication 1, dans lequel le conduit déformable comprend un support métallique encastré dans un matériau en plastique, dans lequel le support métallique est l'un quelconque sélectionné dans le groupe comprenant : une bobine, une hélice et un treillis, et dans lequel le conduit déformable est résilient, et dans lequel le conduit déformable est prévu avec des ondulations (31) pour faciliter l'expansion et/ou la contraction le long de sa longueur et dans lequel le conduit déformable comprend une partie médiane (19) ayant un plus grand diamètre par rapport aux parties d'extrémité (21, 23) opposées.

3. Appareil d'orientation de nébuliseur selon l'une quelconque des revendications 1 à 2, comprenant en outre un connecteur creux (36) monté en rotation par rapport à une partie de sortie (38) du second corps creux (16), dans lequel le mouvement de rotation du connecteur creux par rapport au second corps creux est dans un second plan (β) sensiblement orthogonal au premier plan (α), dans lequel le connecteur creux est monté en rotation par rapport à la partie de sortie du second corps creux via un moyen de fixation (40), et dans lequel le moyen de fixation facilite le mouvement de rotation du connecteur creux par rapport au second corps creux dans le second plan, et dans lequel le moyen de fixation est fourni sous la forme d'un manchon fixant la partie de sortie du second corps creux au connecteur creux tout en fournissant, de manière concomitante, le mouvement de rotation l'un par rapport à l'autre, et dans lequel le connecteur creux met en prise, par couplage, la sortie du second corps creux tout en étant adapté pour fournir, de manière concomitante, le mouvement de rotation du second corps creux par rapport au connecteur creux.

4. Appareil d'orientation de nébuliseur selon l'une quelconque des revendications 1 à 3, dans lequel le premier corps creux (14) est pondéré en étant formé à partir d'un matériau plus lourd que le second corps creux (16), facilitant ainsi le déplacement du premier corps creux sous la force de gravité par rapport au second corps creux.

5. Appareil d'orientation de nébuliseur selon l'une quelconque des revendications 1 à 3, dans lequel les premier et second corps creux sont fabriqués à partir du même matériau, et le premier corps creux est pondéré en étant supérieur en longueur par rapport au second corps creux afin de doter le premier corps creux d'une masse supérieure par rapport au second corps creux, facilitant ainsi le déplacement du premier corps creux sous la force de gravité par rapport au second corps creux.

6. Appareil d'orientation de nébuliseur selon l'une quelconque des revendications 1 à 3, dans lequel le premier corps creux est pondéré en ayant fixé à ce dernier, un moyen de pondération.

7. Appareil d'orientation de nébuliseur selon la revendication 6, dans lequel le moyen de pondération est un collier et/ou une bague entourant au moins partiellement le premier corps creux.

8. Appareil d'orientation de nébuliseur selon la revendication 7, dans lequel le moyen de pondération est formé, de manière solidaire, avec le premier corps creux.

9. Appareil d'orientation de nébuliseur selon la revendication 8, dans lequel le moyen de pondération est un épaississement d'une partie d'une paroi latérale fournissant le premier corps creux.
